# FASCICULE DE BREVET EUROPEEN

(11) **EP 1 498 148 B1**
(45) Date de publication et mention de la délivrance du brevet: **27.08.2008**
(21) Numéro de dépôt: 04291427.5
(22) Date de dépôt: 08.06.2004
(51) Int. Cl.: A61M 1/02

(54) **Système à poches pour le prélèvement et l'échantillonnage d'un fluide biologique d'un donneur**
Beutelsystem zur Entnahme eines Donorfluids und zur Probenentnahme
Bag system for collecting and sampling a donor fluid

(30) Priorité: 20.06.2003 FR 0307493
(43) Date de publication de la demande: 19.01.2005
(73) Titulaire: MACO PHARMA, 59420 Mouvaux (FR)
(72) Inventeur: Goudaliez, Francis, 59155 Faches-Thumesnil (FR); Verpoort, Thierry, 59420 Mouvaux (FR)
(74) Mandataire: Geismar, Thierry

(56) Documents cités:
- EP-A- 1 064 959
- US-B2- 6 387 086

## Description

L'invention concerne un système à poches pour le prélèvement et l'échantillonnage d'un fluide biologique tel que le sang ou un composant sanguin d'un donneur.

De tels systèmes à poches comprennent d'une part une poche de collecte destinée à recevoir le sang prélevé au donneur, en vue d'une transfusion à une autre personne, et d'autre part une poche d'échantillonnage destinée à recevoir les premiers millilitres de sang prélevé. Les poches sont généralement souples, et par exemple constituées de deux feuilles solidarisées l'une à l'autre au voisinage de leur périphérie.

Une première tubulure est connectée à une première extrémité à des moyens de prélèvement, tels qu'une aiguille destinée à être insérée dans le bras du donneur, et à une deuxième extrémité à un orifice d'entrée de la poche de collecte.

Une deuxième tubulure est reliée d'une part à la première tubulure et d'autre part à un orifice d'entrée de la poche d'échantillonnage.

Un système de fermeture, tel qu'un clamp, est placé sur chacune des deux tubulures et permet de diriger le sang prélevé vers la poche d'échantillonnage ou vers la poche de collecte.

Un dispositif d'échantillonnage latéral, relié à l'extérieur de la poche d'échantillonnage à la deuxième tubulure, permet le prélèvement du sang contenu dans la poche d'échantillonnage à l'aide de tubes sous vide. Les échantillons de sang ainsi obtenus sont systématiquement analysés afin de déterminer le groupe Rhésus, de réaliser une numération et de détecter d'éventuelles contaminations comme des virus, des bactéries ou d'autres éléments indésirables présents dans le sang du donneur, et ce avant de transfuser le sang à une autre personne.

Le remplissage de la poche d'échantillonnage préalablement à celui de la poche de collecte présente un certain nombre d'avantages et permet d'améliorer la qualité du prélèvement.

Premièrement, cela permet de diminuer le risque de contamination provenant de la présence de bactéries ou d'autres substances étrangères sur la peau du donneur, puisque les premiers millilitres de sang prélevés présentant éventuellement ces substances étrangères sont envoyés dans la poche d'échantillonnage et non dans la poche de collecte.

Deuxièmement, cela permet de réaliser les échantillons avant que la poche de collecte ne soit totalement remplie, et par conséquent de gagner du temps.

Enfin, lors du prélèvement, la perte de volume de sang pour le donneur étant compensée par le plasma, l'hématocrite du donneur serait plus bas si la poche d'échantillonnage était remplie après la poche de collecte.

On connaît déjà de tels systèmes à poches.

Selon un premier mode de réalisation, la partie extrême de la deuxième tubulure est insérée dans l'orifice d'entrée de la poche d'échantillonnage mais ne pénètre pas - ou du moins très peu - à l'intérieur de ladite poche.

Ceci présente plusieurs inconvénients, dus au fait que, lorsque la poche d'échantillonnage est placée de sorte que son orifice d'entrée soit situé vers le haut, l'extrémité de la deuxième tubulure reliée audit orifice d'entrée se situe au-dessus de l'interface sang - air.

Ainsi, en cas de pression involontaire sur la poche d'échantillonnage, celle-ci se déforme du fait de sa souplesse, et il s'ensuit qu'une partie de l'air contenu dans cette poche remonte dans la tubulure. Puisque la poche d'échantillonnage est reliée aux moyens de prélèvement, il existe un risque non négligeable d'embolie gazeuse, c'est-à-dire d'entrée d'air dans le système veineux du donneur. Or, on sait qu'une entrée d'air en amont du coeur peut entraîner un désamorçage de la pompe cardiaque et un arrêt cardio-circulatoire, donc le décès du donneur.

Même en l'absence de pression involontaire sur la poche d'échantillonnage, cette configuration ne donne pas entière satisfaction puisque, la deuxième tubulure ne plongeant pas dans le sang contenu dans la poche (lorsque la poche est dans la position précitée), il n'est pas possible de remplir des tubes pour l'analyse du sang prélevé pendant le prélèvement lui-même. Il est alors nécessaire, une fois la poche d'échantillonnage remplie, de retourner celle-ci, de façon que l'orifice d'entrée se situe vers le bas. Ceci entraîne une perte de temps non souhaitée.

Selon un deuxième mode de réalisation, la partie extrême de la deuxième tubulure est insérée dans l'orifice d'entrée de la poche d'échantillonnage et pénètre à l'intérieur de ladite poche, de sorte que l'extrémité correspondante de la deuxième tubulure soit située à proximité immédiate du fond de la poche. Ceci est notamment décrit dans le document US 6 387 086.

Cette configuration n'est pas non plus pleinement satisfaisante.

En effet, d'une part, lorsque la poche est orientée de sorte que l'extrémité de la tubulure soit située vers le bas, ladite extrémité est pratiquement dès le début du prélèvement au contact du sang et non de l'air, et les problèmes rencontrés avec le premier mode de réalisation sont évités. Cependant, il est souhaitable de pouvoir placer et utiliser la poche d'échantillonnage dans une position inversée, où l'orifice d'entrée n'est pas située vers le haut mais vers le bas. Dans ce cas, le système du document US 6 387 086 pose les mêmes problèmes que ceux précités.

D'autre part, plus la partie extrême de la deuxième tubulure située à l'intérieur de la poche d'échantillonnage est longue, plus le volume d'air contenu dans ladite poche, avant tout prélèvement, est grand.

Ce volume est la somme :
- du volume contenu dans la partie extrême de la deuxième tubulure située à l'intérieur de la poche d'échantillonnage ;
- et du volume d'air piégé autour de la deuxième tubulure, sur toute sa longueur, entre les deux feuilles formant la poche d'échantillonnage.

Ainsi, plus la longueur de la partie extrême de la deuxième tubulure insérée à l'intérieur de la poche est grande, plus le volume d'air susceptible de remonter dans la tubulure jusqu'à la veine du donneur est important. Le risque d'embolie gazeuse s'en trouve considérablement augmenté.

Il est donc particulièrement important de veiller à limiter au maximum le volume d'air contenu à l'intérieur de la poche avant le prélèvement, et ce d'autant que ce volume est augmenté, sans que l'on puisse l'éviter, du volume d'air initialement contenu dans la partie de la deuxième tubulure située à l'extérieur de la poche d'échantillonnage et qui est chassé vers l'intérieur de la poche pendant le remplissage.

L'invention a pour but de résoudre ces problèmes, en proposant un système à poches pour le prélèvement et l'échantillonnage d'un fluide biologique qui soit efficace et qui permette d'éviter qu'un volume d'air important ne soit réinjecté dans la veine du donneur et ce, quelle que soit la position occupée par la poche d'échantillonnage pendant le prélèvement, et quel que soit le volume de sang contenu dans cette poche.

A cet effet, l'invention concerne un système à poches pour le prélèvement et l'échantillonnage d'un fluide biologique tel que le sang ou un composant sanguin d'un donneur, comprenant :
- une poche de collecte du fluide et une première tubulure connectée à une première extrémité à des moyens de prélèvement et, à une deuxième extrémité, à un orifice d'entrée de la poche de collecte ;
- une poche d'échantillonnage souple définissant un volume intérieur, ledit volume intérieur présentant un côté formant fond et un côté d'introduction opposé audit fond, ledit côté d'introduction présentant un orifice d'entrée ;
- une deuxième tubulure dont une première partie extrême est reliée à la première tubulure à distance de la première extrémité de ladite première tubulure, et dont une deuxième partie extrême est insérée dans l'orifice d'entrée de la poche d'échantillonnage ;
dans lequel la deuxième partie extrême de la deuxième tubulure s'étend à l'intérieur de la poche d'échantillonnage sur une distance comprise entre 25 et 60 % de la distance entre le fond et le côté d'introduction du volume intérieur de la poche d'échantillonnage.

Selon un mode de réalisation possible, la deuxième partie extrême de la deuxième tubulure s'étend à l'intérieur de la poche d'échantillonnage sur une distance comprise entre 30 et 50 % de la distance entre le fond et le côté d'introduction du volume intérieur de la poche d'échantillonnage.

Le système à poches peut comprendre en outre un dispositif d'échantillonnage latéral relié, à l'extérieur de la poche d'échantillonnage, à la deuxième tubulure, et agencé pour permettre le prélèvement d'au moins une partie du fluide contenu dans la poche d'échantillonnage.

Le système à poches peut comporter un ensemble de poches satellites reliées, par l'intermédiaire d'au moins une tubulure, à au moins un orifice de sortie de la poche de collecte.

Par exemple, l'ensemble de poches satellites comprend au moins deux poches et une unité de filtration agencées de sorte à permettre la circulation du fluide en circuit clos.

D'autres objets et avantages de l'invention apparaîtront au cours de la description qui suit, faite en référence aux dessins annexés dans lesquels :
- la figure 1 est une représentation schématique, en élévation, d'un système à poches pour le prélèvement et l'échantillonnage, selon un premier mode de réalisation, comprenant une poche d'échantillonnage munie d'un orifice d'entrée situé vers le haut ;
- la figure 2 est une représentation similaire à celle de la figure 1, l'orifice d'entrée de la poche d'échantillonnage étant situé vers le bas ;
- la figure 3 est une représentation schématique, en élévation, d'un système à poches pour le prélèvement et d'échantillonnage conforme à la figure 1 ou 2, selon un deuxième mode de réalisation.

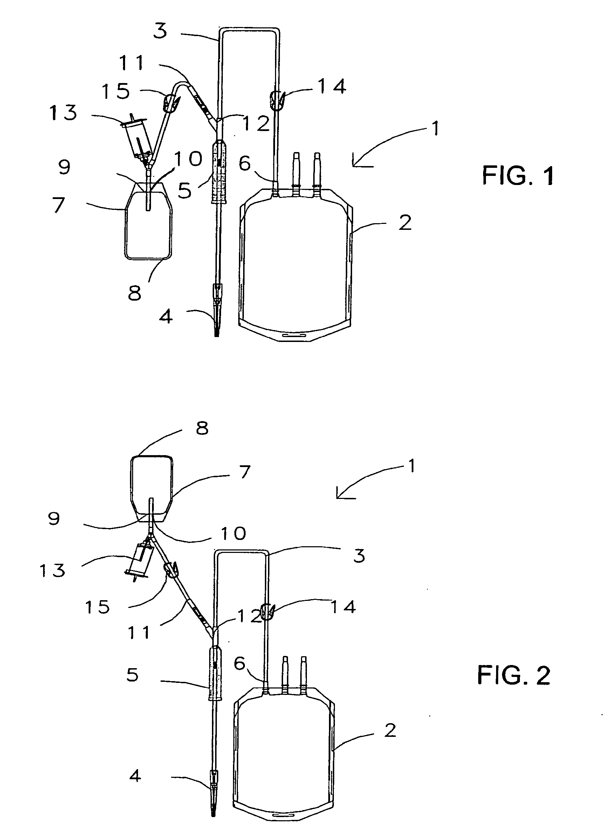

Sur les figures 1 et 2 est représenté un système à poches 1 pour le prélèvement et d'échantillonnage, selon un premier mode de réalisation. Ledit système 1 comprend une poche de collecte 2 du fluide, une première tubulure 3 connectée à une première extrémité à des moyens de prélèvement 4, et à une deuxième extrémité à un orifice d'entrée 6 de la poche de collecte 2. Un protecteur 5 des moyens de prélèvement 4 peut être disposé sur ladite tubulure 3.

Le système 1 comprend en outre une poche d'échantillonnage 7 souple définissant un volume intérieur, ledit volume intérieur présentant un côté 8 formant fond et un côté d'introduction 9 opposé audit fond, ledit côté d'introduction 9 présentant un orifice d'entrée 10.

Le système 1 comprend également une deuxième tubulure 11 dont une première partie extrême est reliée, par l'intermédiaire d'une jonction trois voies 12, à la première tubulure 3, et dont une deuxième partie extrême est insérée dans l'orifice d'entrée 10 de la poche d'échantillonnage 7.

Un dispositif d'échantillonnage 13 latéral est relié, à l'extérieur de la poche d'échantillonnage 7, à la deuxième tubulure 11, et agencé pour permettre le prélèvement d'au moins une partie du fluide contenu dans la poche d'échantillonnage 7.

Ce dispositif d'échantillonnage 13 latéral permet le prélèvement du sang contenu dans la poche d'échantillonnage 7 à l'aide de tubes sous vide.

Des clamps 14, 15 peuvent être placés respectivement sur la première 3 et la deuxième tubulure 11 et permettent de diriger le sang prélevé vers la poche d'échantillonnage 7 ou vers la poche de collecte 2.

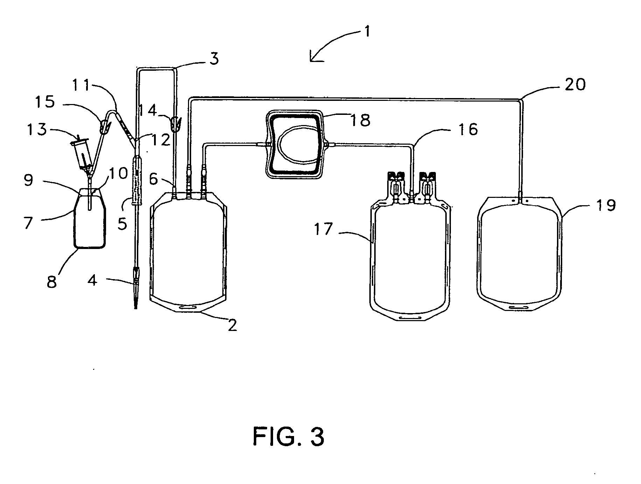

La figure 3 représente un système à poches 1 pour le prélèvement et l'échantillonnage selon un deuxième mode de réalisation.

Afin de réaliser des étapes de filtration et de séparation ainsi que la déleucocytation des différents constituants du sang, la poche de collecte 2 peut être en communication fluidique, par l'intermédiaire d'une troisième tubulure 16, avec une poche satellite 17. Une unité de filtration 18 à déleucocyter est située entre la poche de collecte 2 et la poche satellite 17.

La poche de collecte 2 (et/ou la poche satellite 17) peut être en communication fluidique avec une ou plusieurs autres poches satellites 19 par l'intermédiaire d'une quatrième tubulure 20.

Le système à poches 1 est agencé de sorte à permettre la circulation du fluide en circuit clos.

La deuxième partie extrême de la deuxième tubulure 11 s'étend à l'intérieur de la poche d'échantillonnage 7 sur une distance comprise entre 25 et 60 % de la distance entre le fond 8 et le côté d'introduction 9 du volume intérieur de la poche d'échantillonnage 7.

Selon un mode de réalisation possible, la deuxième partie extrême de la deuxième tubulure 11 s'étend à l'intérieur de la poche d'échantillonnage 7 sur une distance comprise entre 30 et 50 % de la distance entre le fond 8 et le côté d'introduction 9 du volume intérieur de la poche d'échantillonnage 7.

Ainsi, d'une part, la partie extrême de la deuxième tubulure 11 s'étend à l'intérieur de la poche d'échantillonnage 7 sur une distance suffisante.

De ce fait, lorsque l'on utilise la poche d'échantillonnage avec son orifice d'entrée situé vers le haut (comme représenté sur la figure 1), quelque temps après le début du prélèvement, la partie extrême de la deuxième tubulure 11 plonge dans le fluide.

Ceci permet à la fois de limiter considérablement les risques d'embolie gazeuse du donneur suite à une pression involontaire exercée sur la poche d'échantillonnage 7, et d'autoriser le remplissage de tubes pour l'analyse du sang prélevé pendant le prélèvement lui-même.

D'autre part, la partie extrême de la deuxième tubulure 11 ne s'étend pas à l'intérieur de la poche d'échantillonnage 7 sur une distance trop importante.

Ainsi, lorsque l'on utilise la poche d'échantillonnage avec son orifice d'entrée situé vers le bas (comme représenté sur la figure 2), quelque temps après le début du prélèvement, la partie extrême de la deuxième tubulure 11 plonge dans le fluide. Comme indiqué précédemment, ceci permet de réduire le risque d'embolie gazeuse pour le donneur mais également de remplir rapidement les tubes pour l'analyse du sang prélevé.

En outre, la longueur de la deuxième tubulure 11 située à l'intérieur de la poche d'échantillonnage 7 étant réduite, le volume d'air contenu dans ladite poche 7, avant tout prélèvement, est également réduit.

Le volume de gaz étant un paramètre essentiel dans l'apparition d'une embolie gazeuse, le risque d'embolie gazeuse s'en trouve considérablement diminué.

Le tableau ci-dessous met en évidence la relation entre le volume de gaz susceptible de remonter dans la tubulure 11 jusqu'à la veine du donneur et la longueur de la portion de tubulure 11 s'étendant au sein de la poche d'échantillonnage 7.

| | Longueur tubulure (cm) | Volume d'air (ml) | Longueur tubulure (cm) | Volume d'air (ml) | Longueur tubulure (cm) | Volume d'air (ml) |
|---|---|---|---|---|---|---|
| Essai n°1 | 2 | 5 | 5 | 8 | 10 | 10 |
| Essai n°2 | 2 | 6 | 5 | 8 | 10 | 11 |
| Essai n°3 | 2 | 6 | 5 | 8 | 10 | 11 |
| Essai n°4 | 2 | 5 | 5 | 8 | 10 | 11 |
| Moyenne | 2 | 5,5 | 5 | 8 | 10 | 10,75 |

Les longueurs de tubulure 2, 5 et 10 cm correspondent respectivement à un pourcentage de 19, 48 et 95 % par rapport à la longueur de la poche 7 utilisée dans ces tests.

Les caractéristiques de cette poche sont les suivantes :
- longueur : 10,5 cm ;
- largeur : 5 cm ;
- volume : 40 ml.

Si d'une part, l'on veut éviter les inconvénients apparaissant lorsque la tubulure 11 ne pénètre pas ou du moins très peu au sein de ladite poche 7, et d'autre part limiter le volume d'air contenu dans la poche 7, il convient que la partie extrême de la deuxième tubulure 11 s'étende à l'intérieur de la poche d'échantillonnage 7 sur une distance comprise entre 25 et 60 % de la distance entre le fond 8 et le côté d'introduction 9 du volume intérieur de la poche d'échantillonnage 7.

Ainsi, l'invention permet de limiter considérablement les risques d'embolie gazeuse quelle que soit la position dans laquelle la poche d'échantillonnage 7 est utilisée, à savoir en particulier l'une des deux positions extrêmes représentées sur les figures 1 et 2.

## Revendications

1. Système à poches pour le prélèvement et l'échantillonnage d'un fluide biologique tel que le sang ou un composant sanguin d'un donneur, comprenant :
- une poche de collecte (2) du fluide et une première tubulure (3) connectée à une première extrémité à des moyens de prélèvement (4) et, à une deuxième extrémité, à un orifice d'entrée (6) de la poche de collecte (2) ;
- une poche d'échantillonnage (7) souple définissant un volume intérieur, ledit volume intérieur présentant un côté formant fond (8) et un côté d'introduction (9) opposé audit fond (8), ledit côté d'introduction (9) présentant un orifice d'entrée (10) ;
- une deuxième tubulure (11) dont une première partie extrême est reliée à la première tubulure (3) à distance de la première extrémité de ladite première tubulure (3), et dont une deuxième partie extrême est insérée dans l'orifice d'entrée (10) de la poche d'échantillonnage (7) ;
**caractérisé en ce que** la deuxième partie extrême de la deuxième tubulure (11) s'étend à l'intérieur de la poche d'échantillonnage (7) sur une distance comprise entre 25 et 60 % de la distance entre le fond (8) et le côté d'introduction (9) du volume intérieur de la poche d'échantillonnage (7).

2. Système à poches selon la revendication 1, **caractérisé en ce que** la deuxième partie extrême de la deuxième tubulure (11) s'étend à l'intérieur de la poche d'échantillonnage (7) sur une distance comprise entre 30 et 50 % de la distance entre le fond (8) et le côté d'introduction (9) du volume intérieur de la poche d'échantillonnage (7).

3. Système à poches selon la revendication 1 ou 2, **caractérisé en ce qu'**il comprend en outre un dispositif d'échantillonnage (13) latéral relié, à l'extérieur de la poche d'échantillonnage (7), à la deuxième tubulure (11), et agencé pour permettre le prélèvement d'au moins une partie du fluide contenu dans la poche d'échantillonnage (7).

4. Système à poches selon l'une quelconque des revendications 1 à 3, **caractérisé en ce qu'**il comporte en outre un ensemble de poches satellites (17, 19) reliées, par l'intermédiaire d'au moins une tubulure (16, 20), à au moins un orifice de sortie de la poche de collecte (2).

5. Système à poches selon la revendication 4, **caractérisé en ce que** l'ensemble de poches satellites comprend au moins deux poches (17, 19) et une unité de filtration (18) agencées de sorte à permettre la circulation du fluide en circuit clos.

## Claims

1. A system of bags for the collection and the sampling of a body fluid such as blood or a blood compound from a donor, comprising:
- a bag (2) for collecting the fluid and a first tube (3) connected at a first end to sampling means (4) and at a second end, to an inlet (6) to the collection bag (2);
- a flexible sampling bag (7) defining an internal volume, said internal volume having a side forming the bottom (8) and an introduction side (9) opposite said bottom (8), said introduction side (9) having an inlet (10);
- a second tube (11), a first end part of which is connected to the first tube (3) at a distance from the first end of said first tube (3), and a second end part of which is inserted in the inlet (10) of the sampling bag (7);
**characterized in that** the second end part of the second tube (11) extends inside the sampling bag (7) on a distance between 25 and 60% of the distance between the bottom (8) and the introduction side (9) of the internal volume of the sampling bag (7).

2. A system of bags according to claim 1, **characterized in that** the second end part of the second tube (11) extends inside the sampling bag (7) on a distance between 30 and 50% of the distance between the bottom (8) and the introduction side (9) of the internal volume of the sampling bag (7).

3. A system of bags according to claim 1 or 2, **characterized in that** it further includes a side sampling device (13) connected, outside the sampling bag (7), to the second tube (11) and so arranged as to allow the sampling of at least a part of the fluid contained in the sampling bag (7).

4. A system of bags according to any one of claims 1 to 3, **characterized in that** it further includes a set of satellite bags (17, 19) connected to at least one outlet of the collection bag (2) through at least one tube (16, 20).

5. A system of bags according to claim 4, **characterized in that** the set of satellite bags comprises at least two bags (17, 19) and a filtration unit (18) so arranged as to allow the circulation of the fluid in closed circuit.

## Patentansprüche

1. Beutelsystem für die Entnahme und Probenahme einer biologischen Flüssigkeit wie zum Beispiel Blut oder einen Blutbestandteil eines Spenders, das folgende Teile umfaßt:
- Einen Sammelbeutel (2) für die Flüssigkeit und einen ersten Stutzen (3), der an einem ersten Ende an Mittel zur Entnahme (4) und an einem zweiten Ende an eine Eintrittsöffnung (6) des Sammelbeutels (2) angeschlossen ist;
- Einen weichen Probenbeutel (7), der ein Innenvolumen definiert, wobei das besagte Innenvolumen eine einen Boden (8) bildende Seite aufweist und eine Eintrittsseite (9) entgegengesetzt zum besagten Boden (8), wobei die besagte Eintrittsseite (9) eine Eintrittsöffnung (10) aufweist;
- Einen zweiten Stutzen (11), dessen erster Endteil mit dem ersten Stutzen (3) in Entfernung vom ersten Ende des besagten ersten Stutzens (3) verbunden ist, und dessen zweiter Endteil in die Eintrittsöffnung (10) des Probenbeutels (7) eingesetzt ist;
**Dadurch gekennzeichnet, daß** sich der zweite Endteil des zweiten Stutzens (11) im Innern des Probenbeutels (7) über eine Entfernung von zwischen 25 und 60% des Abstands zwischen dem Boden (8) und der Eintrittsseite (9) des Innenvolumens des Probenbeutels (7) erstreckt.

2. Beutelsystem nach Anspruch 1, **dadurch gekennzeichnet, daß** sich der zweite Endteil des zweiten Stutzens (11) im Innern des Probenbeutels (7) über eine Entfernung von zwischen 30 und 50% des Abstands zwischen dem Boden (8) und der Eintrittsseite (9) des Innenvolumens des Probenbeutels (7) erstreckt.

3. Beutelsystem nach Anspruch 1 oder 2, **dadurch gekennzeichnet, daß** es ferner eine seitliche Probenahme-Vorrichtung (13) umfaßt, die außerhalb des Probenbeutels (7) mit dem zweiten Stutzen (11) verbunden und so gestaltet ist, daß die Entnahme mindestens eines Teils der im Probenbeutel (7) enthaltenen Flüssigkeit möglich ist.

4. Beutelsystem nach einem beliebigen der vorstehenden Ansprüche 1 bis 3, **dadurch gekennzeichnet, daß** es ferner eine Reihe von Satellitenbeuteln (17, 19) umfaßt, die vermittels mindestens eines Stutzens (16, 20) mit mindestens einer Austrittsöffnung des Sammelbeutels (2) verbunden sind.

5. Beutelsystem nach Anspruch 4, **dadurch gekennzeichnet, daß** die Satellitenbeutel mindestens zwei Beutel (17, 19) und eine Filtriereinrichtung (18) umfassen, die so angeordnet sind, daß der Umlauf der Flüssigkeit im geschlossenen Kreislauf möglich ist.
